(19) Europäisches Patentamt
European Patent Office
Office européen des brevets

(11) Veröffentlichungsnummer : **0 023 584**
**B1**

(12) # EUROPÄISCHE PATENTSCHRIFT

(45) Veröffentlichungstag der Patentschrift : **26.01.83**

(21) Anmeldenummer : **80103850.6**

(22) Anmeldetag : **07.07.80**

(51) Int. Cl.³ : **C 07 C 17/32**, C 07 C 21/24, C 07 C 51/58, C 07 C 63/36, C 07 C 79/12, C 07 C143/70

(54) **Verfahren zur Herstellung von Trifluormethyl-Naphthalinen.**

(30) Priorität : **17.07.79 DE 2928745**

(43) Veröffentlichungstag der Anmeldung : **11.02.81 Patentblatt 81/06**

(45) Bekanntmachung des Hinweises auf die Patenterteilung : **26.01.83 Patentblatt 83/04**

(84) Benannte Vertragsstaaten : **BE CH DE FR GB IT LI**

(56) Entgegenhaltungen :
**EP A 0 008 453**
**US A 2 273 922**

**Chemical Abstracts Band 86, Nr. 19, 9 Mai 1977 Columbus, Ohio, USA H. HOSOKAWA et al. « Synthesis of (trifluoromethyl) naphthalenes » Seiten 527, rechte Spalte und 528, linke Spalte, Abstract Nr. 139662m.**
**Chemical Abstracts Band 76, Nr. 19, 8 Mai 1972 Columbus, Ohio, USA K. HOSOKAWA et al. « Synthesis of beta-trifluoromethylnaphthalene derivatives » Seite 463, linke Spalte, Abstract Nr. 112946p.**
**Chemical Abstracts Band 77, Nr. 3, 17 Juli 1972 Columbus, Ohio, USA B. V. KUNSHENKO et al. « alpha and beta-trihalomethyl derivatives of naphthalene » Seite 490, linke Spalte, Abstract Nr. 19423b.**

(73) Patentinhaber : **BAYER AG**
**Zentralbereich Patente, Marken und Lizenzen**
**D-5090 Leverkusen 1, Bayerwerk (DE)**

(72) Erfinder : **Marhold, Albrecht, Dr.**
**Carl-Duisberg-Strasse 329**
**D-5090 Leverkusen 1 (DE)**
Erfinder : **Klauke, Erich, Dr.**
**Eichendorffweg 8**
**D-5068 Odenthal (DE)**

**EP 0 023 584 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von Trifluormethyl-Naphthalinen

Die Erfindung betrifft ein Verfahren zur Herstellung von gegebenenfalls substituierten Trifluormethyl-Naphthalinen.

Aus US 2.432.393 ist bekannt, 1-Trifluormethylnaphthalin durch Reaktion von 1-Trichlormethylnaphthalin mit Antimontrifluorid herzustellen. Der Nachteil dieses Verfahrens liegt darin, daß das als Vorprodukt benötigte 1-Trichlormethyl-naphthalin nicht einfach aus Methylnaphthalin durch Chlorierung zugänglich ist, da die Umsetzung von Methylnaphthalin mit Chlor in großem Umfange zu kernchlorierten Produkten führt (Chem. Ber. *24,* 3921 (1891)). Aus Zh. Org. Khim. (englische Übersetzung) *8,* 838 (1972) ist es bekannt, die an den Naphthalinkern gebundene Carboxylgruppe durch Umsetzung mit Schwefeltetrafluorid in die Trifluormethylgruppe umzuwandeln. Im Falle der nicht-substituierten 1-Naphthalincarbonsäure ergibt dieses Verfahren eine Ausbeute von 7 %. Weiterhin ist es aus US 2.273.922 bekannt, Naphthalin mit Tetrachlorkohlenstoff und Fluorwasserstoff in Gegenwart von Kupfer bei 150 bis 155 °C in einer 48-stündigen Reaktion umzusetzen. Bei diesem Verfahren soll laut Anspruch Trifluormethyl-naphthalin erhalten werden. In dieser US 2.273.922 werden zwei Reaktionsprodukte beschrieben, von denen eines ein Molekulargewicht von über 500 haben soll und dessen Konstitution nicht näher beschrieben ist, während das andere ein Mono-trifluor-methyl-naphthalin mit einem Schmelzpunkt von 92 °C sein soll. Nach den eigenen Beobachtungen trifft dieser Schmelzpunkt für das 1-Trifluormethyl-naphthalin oder für das 2-Trifluormethyl-naphthalin auch nicht annähernd zu.

Es wurde ein Verfahren zur Herstellung von gegebenenfalls substituierten Trifluormethyl-naphthalinen durch Umsetzung von Naphthalinen mit Tetrachlorkohlenstoff und Fluorwasserstoff und unter Druck gefunden, das dadurch gekennzeichnet ist, daß man eine Verbindung der Formel

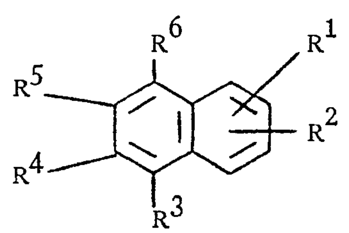

(I)

in der

$R^1$ Wasserstoff, Alkyl, Aralkyl, Aryl, Aryloxy, Arylthio, Polyhalogenalkoxy, Polyhalogenalkylthio, Halogen, Nitro, Halogencarbonyl, Halogensulfonyl, Alkylsulfonyl oder Arylsulfonyl bedeutet, wobei die in den Substituenten $R^1$ enthaltenen aromatischen Kerne ihrerseits durch Halogen, Alkyl, Polyhalogenalkyloxy oder Polyhalogenalkylthio substituiert sein können,

$R^2$ für Wasserstoff, Halogen oder Alkyl steht und

$R^3$, $R^4$, $R^5$ und $R^6$ Wasserstoff bedeuten oder

$R^3$ und $R^4$ oder $R^4$ und $R^5$ jeweils gemeinsam einen kondensierten aromatischen Ring bedeuten, wobei für den Fall, daß $R^3$ und $R^4$ gemeinsam einen kondensierten aromatischen Ring bilden, $R^5$ und $R^6$ unabhängig voneinander außer Wasserstoff auch noch Halogen, Nitro, Halogencarbonyl oder Halogensulfonyl bedeuten können und wobei für den Fall, daß $R^4$ und $R^5$ gemeinsam einen kondensierten aromatischen Ring bilden, $R^3$ und $R^6$ unabhängig voneinander außer Wasserstoff auch noch Halogen, Nitro, Halogencarbonyl oder Halogensulfonyl bedeuten können einsetzt, die Umsetzung unter Ausschluß von Kupfer bei einer Temperatur von 70 bis 120 °C und bei einem Druck von 5 bis 30 bar durchführt und das Reaktionsprodukt gegebenenfalls anschließend mit katalytisch angeregtem Wasserstoff behandelt.

Als Alkyl seien beispielsweise geradkettige oder verzweigte Kohlenwasserstoffreste mit 1 bis 8 Kohlenstoffatomen genannt, wie Methyl, Ethyl, Propyl, Butyl, Hexyl oder Octyl, bevorzugt solche mit 1 bis 4 Kohlenstoffatomen, ganz bevorzugt Methyl.

Als Aralkyl seien beispielsweise Kohlenwasserstoffreste mit 1 oder 2 Kohlenstoffatomen im aliphatischen Teil und 6 bis 12 Kohlenstoffatomen im aromatischen Teil genannt, wie Benzyl, Phenylethyl, Diphenylmethyl, Diphenylethyl oder Naphthylmethyl, bevorzugt Benzyl.

Als Aryl seien beispielsweise aromatischen Kohlenwasserstoffreste mit 6 bis 12 Kohlenstoffatomen genannt, wie Phenyl, Diphenyl oder Naphthyl, bevorzugt Phenyl.

Als Aryloxy seien beispielsweise Reste mit 6 bis 12 Kohlenstoffatomen genannt, wie Phenyloxy, Diphenyloxy oder Naphthyloxy, bevorzugt Phenyloxy.

Als Arylthio seien beispielsweise Reste mit 6 bis 12 Kohlenstoffatomen genannt, wie Phenylthio, Diphenylthio oder Naphthylthio, bevorzugt Phenylthio.

Als Polyhalogenalkoxy seinen beispielsweise durch Fluor, Chlor oder Brom substituierte Reste mit 1 bis 4 Kohlenstoffatomen genannt, wie Trifluormethyloxy, Trichlormethyloxy, Tribrommethyloxy, Difluorchlormethyloxy, Difluorbrommethyloxy, Fluordichlormethyloxy, Fluordibrommethyloxy, Dibromchlormethyloxy, Bromdichlormethyloxy, Pentafluorethyloxy, Pentachlorethyloxy, Pentabromethyloxy, α-Difluor-β-fluor-β-chlor-ethyloxy, die gleichartig oder verschiedenartig halogenierten Propyloxyreste oder

die gleichartig oder verschiedenartig halogenierten Butyloxyreste genannt. Bevorzugt sind die Polyhalogenalkoxy-Reste mit 1 bis 2 Kohlenstoffatomen, ganz besonders bevorzugt sind die gleichartig oder verschiedenartig halogenierten Methoxy-Reste.

Als Polyhalogenalkylthio seien beispielsweise solche genannt, wie sie als Polyhalogenalkoxyreste genannt sind, wobei jedoch anstelle des Sauerstoffatoms ein Schwefelatom tritt. Sie können 1 bis 4 Kohlenstoffatome, bevorzugt 1 bis 2 Kohlenstoffatome oder ganz bevorzugt 1 Kohlenstoffatom haben.

Als Halogen sei beispielsweise Fluor, Chlor, Brom oder Jod, bevorzugt Fluor, Chlor oder Brom, ganz besonders bevorzugt Chlor, genannt.

Als Halogencarbonyl seien beispielsweise Fluorcarbonyl, Chlorcarbonyl oder Bromcarbonyl, bevorzugt Chlorcarbonyl genannt.

Als Halogensulfonyl seien beispielsweise Fluorsulfonyl, Chlorsulfonyl oder Bromsulfonyl, bevorzugt Chlorsulfonyl, genannt.

Als Alkylsulfonyl seien beispielsweise Sulfonylreste mit $C_1$-$C_4$-Alkylgruppen genannt, wie Methylsulfonyl, Ethylsulfonyl, Propylsulfonyl, Isopropylsulfonyl, Butylsulfonyl oder Isobutylsulfonyl, bevorzugt Methylsulfonyl und Ethylsulfonyl.

Als Arylsulfonyl seien beispielsweise Sulfonylgruppen mit $C_6$ bis $C_{12}$-Arylgruppen genannt, wie Phenylsulfonyl, Diphenyl-sulfonyl, Naphthylsulfonyl, bevorzugt Phenylsulfonyl.

Die in den Substituenten enthaltenen aromatischen Kerne können ihrerseits durch Halogen, $C_1$-$C_4$-Alkyl, $C_1$-$C_4$-Polyhalogenalkyloxy oder $C_1$-$C_4$-Polyhalogenalkylthio substituiert sein.

Die Reste $R_3$ und $R_4$ oder $R_4$ und $R_5$ können jeweils gemeinsam einen kondensierten aromatischen Ring bedeuten, so daß Verbindungen der Phenanthren- bzw. Anthracen-Reihe entstehen können.

Bevorzugte Verbindungen im Rahmen der Formel (I) sind Verbindungen der Formel

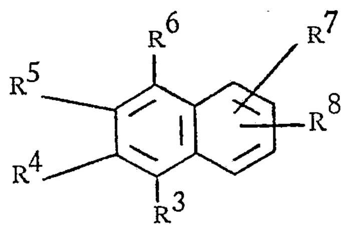

(II)

in der

$R^7$ Aryloxy, Arylthio, Polyhalogenalkoxy, Polyhalogenalkylthio, Halogen, Nitro, Halogencarbonyl, Halogensulfonyl, Alkylsulfonyl oder Arylsulfonyl bedeutet, wobei die im Substituenten $R^7$ enthaltenen aromatischen Kerne ihrerseits durch Halogen, Alkyl, Polyhalogenalkyloxy oder Polyhalogenalkylthio substituiert sein können,

$R^8$ für Wasserstoff oder Halogen steht und

$R^3$ bis $R^6$ die oben angegebene Bedeutung haben.

Besonders bevorzugte Verbindungen im Rahmen der Formel (I) sind Naphthaline der Formel

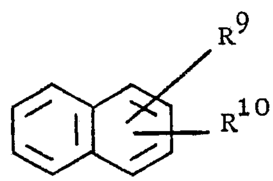

(III)

in der

$R^9$ Halogen, Nitro, Halogencarbonyl oder Halogensulfonyl bedeutet und

$R^{10}$ für Wasserstoff oder Halogen steht.

Ganz besonders bevorzugte Verbindungen im Rahmen der Formel (I) sind Naphthaline der Formel

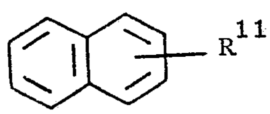

in der

$R^{11}$ Chlorcarbonyl oder Chlorsulfonyl bedeutet.

Als Beispiele für Verbindungen der Formeln (I, (II) und (III) seien genannt:

Naphthalin, 1-Chlornaphthalin, 2-Chlornaphthalin, 1-Naphthalincarbonsäurechlorid, 2-Naphthalincarbonsäurechlorid, 1-Naphthalinsulfonsäurechlorid, 2-Naphthalinsulfonsäurechlorid, 1-Nitronaphthalin, 2-Nitronaphthalin, 1,4-Dichlornaphthalin, 1-Nitro-4-methylnaphthalin, 1-Nitro-2,3-dimethylnaphthalin, 1-Nitro-2,6-dimethylnaphthalin, 1-Bromnaphthalin, 1-Methyl-4-chlorsulfonyl-naphthalin, 1-Chlor-4-methylnaphthalin, 9,10-Dichloranthracen, 1-Bromnaphthalin, 2-Bromnaphthalin, 1-Methylnaphthalin, 1-Ethylnaphthalin, 1-Butylnaphthalin, 2-Methylnaphthalin, 2-Ethylnaphthalin, 2-Propylnaphthalin, 1-Trichlormethoxynaphthalin, 1-Pentabromethylthio-naphthalin.

Tetrachlorkohlenstoff wird im erfindungsgemäßen Verfahren beispielsweise in einer Menge von 3 bis 20 Mol, bevorzugt 6 bis 12 Mol pro Mol Verbindung der Formel (I) eingesetzt.

Fluorwasserstoff wird im erfindungsgemäßen Verfahren beispielsweise in einer Menge von 3 bis 60 Mol, bevorzugt 5 bis 50 Mol pro Mol Verbindung der Formel (I) eingesetzt.

Werden Tetrachlorkohlenstoff oder Fluorwasserstoff in kleineren als den angegebenen Mengen eingesetzt, besteht die Gefahr einer Ausbeuteverminderung durch unvollständige Reaktion. Der Einsatz einer größeren Menge von Tetrachlorkohlenstoff oder Fluorwasserstoff als der angegebenen Menge ist aus wirtschaftlichen Gründen wenig sinnvoll.

Das erfindungsgemäße Verfahren wird beispielsweise bei einer Temperatur von 70 bis 120 °C, bevorzugt von 80 bis 120 °C, besonders bevorzugt von 90 bis 110 °C durchgeführt.

Das erfindungsgemäße Verfahren wird bei erhöhtem Druck durchgeführt. Als solcher sei beispielsweise ein Druck von 5 bis 30 bar, bevorzugt 10 bis 20 bar genannt. Dieser Druck kann beispielsweise durch den Eigendruck der eingesetzten und der entstehenden Reaktionspartner erreicht werden. Es ist jedoch auch möglich, den gewählten Druck durch komprimierte atmosphärische Luft oder durch ein komprimiertes Inertgas, beispielsweise Stickstoff, einzustellen.

Für den Fall, daß durch die erfindungsgemäße Umsetzung einer Verbindung der Formel (I) mit Tetrachlorkohlenstoff und Fluorwasserstoff mehrere isomere Reaktionsprodukte entstehen, kann es günstig sein, den oder die ursprünglich in der Verbindung der Formel (I) vorhandenen Substituenten durch Umsetzung mit katalytisch angeregtem Wasserstoff in Gegenwart einer alkalisch reagierenden Substanz ganz oder teilweise zu entfernen, um so die Zahl der isomeren, gegebenenfalls substituierten Trifluormethyl-naphthaline zu verringern.

Bei dieser Variante des erfindungsgemäßen Verfahrens werden bevorzugt Verbindungen der Formel

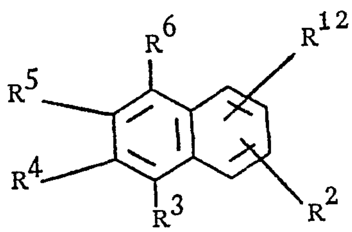

(IV)

in der

$R^2$ bis $R^6$ die obengenannte Bedeutung haben und

$R^{12}$ Chlor oder Brom bedeutet, eingesetzt.

So können beispielsweise bei der erfindungsgemäßen Umsetzung von 1-Chlor-naphthalin entsprechend der Formelgleichung

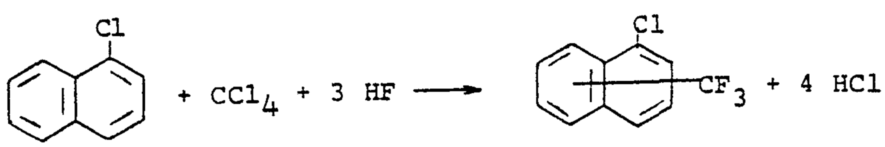

das 1-Chlor-4-trifluormethyl-naphthalin und/oder das 1-Chlor-5-trifluormethyl-naphthalin und/oder das 1-Chlor-8-trifluormethyl-naphthalin als Hauptprodukte entstehen. Durch eine Dechlorierung der genannten Isomeren, die erfindungsgemäß durch Behandlung mit katalytisch angeregtem Wasserstoff in Gegenwart einer alkalisch reagierenden Substanz erfolgt, erhält man einheitliches 1-Trifluormethyl-naphthalin in hoher Ausbeute.

Zur katalytischen Anregung des Wasserstoffs seien beispielsweise Katalysatoren vom Raney-Typ, wie Raney-Nickel, Raney-Eisen-Nickel, Raney-Kupfer-Nickel oder Raney-Kobalt-Nickel, genannt. Bevorzugt ist die Verwendung von Raney-Nickel.

Die Umsetzung mit katalytisch angeregtem Wasserstoff wird in einem für katalytische Hydrierungen geeigneten Lösungsmittel, beispielsweise in Methanol, Ethanol oder Dioxan, durchgeführt. Als Temperatur für die Hydrierung sei beispielsweise 20 bis 60 °C, bevorzugt 30 bis 50 °C, genannt.

Für die erfindungsgemäße Umsetzung mit katalytisch angeregtem Wasserstoff sei beispielsweise ein Wasserstoffdruck von 25 bis 70 bar, bevorzugt 30 bis 50 bar genannt.

Die erfindungsgemäße Hydrierung wird in Gegenwart einer alkalisch reagierenden Verbindung, wie Natriumhydroxid, Kaliumhydroxid, Natriumcarbonat, Kaliumcarbonat, Natriumhydrogencarbonat, Kaliumhydrogencarbonat, bevorzugt Natriumhydroxid oder Kaliumhydroxid, durchgeführt. Die alkalisch reagierende Substanz wird in einer Menge von 1,1 bis 3, bevorzugt 1,2 bis 2 Äquivalenten pro Äquivalent des vom Trifluormethyl-naphthalin zu entfernenden Substituenten eingesetzt.

Mit Hilfe des erfindungsgemäßen Verfahrens können Verbindungen der Formel

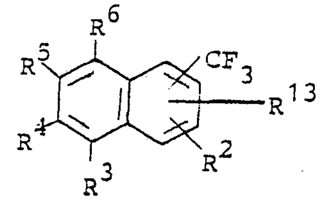

(IV)

in der

$R^2$ bis $R^6$ die oben angegebene Bedeutung haben und

$R^{13}$ Wasserstoff, Alkyl, Aralkyl, Aryl, Aryloxy, Arylthio, Polyfluoralkoxy, Polyfluoralkylthio, Halogen, Nitro, Fluorcarbonyl, Fluorsulfonyl, Alkylsulfonyl oder Arylsulfonyl bedeutet, wobei die im Substituenten $R^{13}$ enthaltenen aromatischen Kern ihrerseits durch Halogen, Alkyl, Polyfluoralkoxy oder Polyfluoralkylthio substituiert sein können, hergestellt werden.

Beispiele für Verbindungen der Formel (IV) sind :

1-Trifluormethylnaphthalin, 2-Trifluormethylnaphthalin, 5-Trifluormethyl-naphthalin-1-carbonsäurefluorid, 5-Trifluormethyl-naphthalin-2-carbonsäurefluorid, 5-Nitro-1-trifluormethylnaphthalin, 5-Trifluormethylnaphthalin-1-sulfonsäurefluorid, 5-Trifluormethylnaphthalin-2-sulfonsäurefluorid, 5-Trifluormethyl-1-methylsulfonyl-naphthalin, 1-Trifluormethyl-9.10-dichlor-anthracen.

Das erfindungsgemäße Verfahren kann beispielsweise wie folgt durchgeführt werden :

In einem Autoklaven aus rostfreiem Stahl wird wasserfreier flüssiger Fluorwasserstoff unter Kühlung vorgelegt und eine Lösung des Naphthalins der Formel (I) in Tetrachlorkohlenstoff zugegeben. Anschließend wird mit Stickstoff ein Druck von etwa 3 bar eingestellt und der Inhalt des Autoklavens unter Rühren auf die gewünschte Reaktionstemperatur gebracht. Nach kurzer Zeit zeigt sich der Beginn der Reaktion am Ansteigen des Druckes durch den entstehenden Chlorwasserstoff. Dieser Chlorwasserstoff wird über einen mit Sole gekühlten Rückflußkühler mit Regelventil kontinuierlich oder absatzweise entspannt. Nach Abschluß der Reaktion werden der überschüssige Fluorwasserstoff und der überschüssige Tetrachlorkohlenstoff abdestilliert und das Reaktionsprodukt durch an sich bekannte Methoden, beispielsweise durch Destillation oder Kristallisation, isoliert.

Für den Fall, daß eine weitere Umsetzung des Reaktionsproduktes mit katalytisch angeregtem Wasserstoff vorgesehen ist, wird das rohe Reaktionsprodukt, gelöst in einem geeigneten Lösungsmittel, beispielsweise Methanol, in einer Hydrierapparatur vorgelegt. Die alkalisch reagierende Substanz und der Raney-Katalysator werden zugegeben und bei einer Reaktionstemperatur von etwa 30 bis 50 °C wird ein Wasserstoffdruck von etwa 30 bis 50 bar bis zur Sättigung aufrechterhalten. Nach Beendigung der Hydrierung wird das Reaktionsgemisch in Wasser eingerührt, der Katalysator abfiltriert und das Reaktionsprodukt aus der abgeschiedenen organischen Phase durch übliche Aufarbeitungsmethoden, beispielsweise Kristallisation oder Destillation, aufgearbeitet.

Das erfindungsgemäße Verfahren ermöglicht in technisch fortschrittlicher Weise die Einführung der Trifluormethylgruppe in Naphthalin-Derivate in nur einem Reaktionsschritt und mit hoher Ausbeute, während die Verfahren nach dem Stand der Technik mehrstufig sind oder unklar definierte Produkte liefern.

Die glatte Ausführungsmöglichkeit des erfindungsgemäßen Verfahrens ist überraschend, da erwartet werden konnte, daß Naphthalin-Derivate in Gegenwart einer Chloralkylverbindung wie Tetrachlorkohlenstoff und in Gegenwart eines Friedel-Crafts-Katalysators wie Fluorwasserstoff hauptsächlich unkontrollierte Kondensations- und Verharzungs-Reaktionen eingehen würde, wie dies für die Reaktion von Benzol mit Tetrachlorkohlenstoff in Gegenwart von Aluminiumchlorid aus Organicum, Organischchemisches Grundpraktikum, 8. Auflage, VEB-Verlag, Berlin 1968, Seite 302, bekannt ist.

Die nach dem erfindungsgemäßen Verfahren herstellbaren gegebenenfalls substituierten Trifluormethyl-naphthaline sind wichtige Zwischenprodukte für Farbstoffe und Insektizide. So kann beispielsweise Nitro-trifluormethyl-naphthalin zum Amino-trifluormethyl-naphthalin reduziert werden. Durch Diazotieren des letzteren und Kuppeln mit Dimethylanilin erhält man einen Farbstoff dessen Absorptionsmaximum im Vergleich zum analogen, jedoch vom p-Amino-benzotrifluorid abgeleiteten Farbstoff in den längerwelligen Spektralbereich verschoben ist (Zh. Org. Khim. (engl. Übersetzung) 8, 838 (1972)).

## Beispiel 1

In einem 5 l Rührautoklaven aus Edelstahl werden bei 10 °C 1 600 ml wasserfreier Fluorwasserstoff vorgelegt und anschließend 750 g 1-Chlor-naphthalin, gelöst in 1 900 ml Tetrachlorkohlenstoff, zugegeben. Nun werden etwa 3 bar Stickstoff aufgedrückt, und die Reaktionsmischung wird unter Rühren auf eine Temperatur von 100 bis 105 °C erhitzt. Es entwickelt sich Chlorwasserstoff, der über einen auf – 10 °C gekühlten Rückflußkühler und über ein Regelventil bei 16 bar entspannt wird. Man läßt die Reaktion 8 Stunden unter diesen Bedingungen ablaufen und kühlt dann ab. Nach dem Entspannen wird aus dem Reaktionsansatz zunächst der überschüssige Fluorwasserstoff und dann der überschüssige Tetrachlorkohlenstoff abdestilliert. Durch Destillation unter vermindertem Druck erhält man 807 g Isomerenmischung von 1-Chlor-trifluormethyl-naphthalin. Kp. : 120 bis 130 °C/15 mm Hg (das entspricht 20 mbar) ; $n_D^{20}$ : 1.554 0.

200 g der isomeren 1-Chlor-trifluormethyl-naphthaline werden in einer Hydrierapparatur, gelöst in 800 ml Methanol, vorgelegt. 52 g Natriumhydroxid und 20 g Raney-Nickel werden zugegeben, und der Reaktionsansatz wird auf 40 °C erwärmt. Dann hydriert man bei einem Wasserstoffdruck von 40 bar, bis Sättigung an Wasserstoff eingetreten ist. Nach dem Abkühlen und Entspannen wird die Reaktionsmischung in 500 ml Wasser eingerührt, der Katalysator abfiltriert und die ausgeschiedene organische Phase abgetrennt. Nach der Trocknung der organischen Phase über Natriumsulfat und einer Destillation bei vermindertem Druck erhält man 147 g des bei Raumtemperatur flüssigen 1-Trifluormethyl-naphthalins ; Kp. : 102 bis 104 °C/16 mm Hg (das entspricht 21, 3 mbar) ; $n_D^{20}$ : 1.536 2.

Beispiel 2

500 ml Fluorwasserstoff, 600 ml Tetrachlorkohlenstoff und 128 g Naphthalin werden, wie in Beispiel 1 beschrieben, 4 Stunden bei 105 °C und 15 bar zur Reaktion gebracht. Nach destillativer Aufarbeitung erhält man 50 g eines Produktes vom Kp. : 70 bis 80 °C/3 mm Hg (das entspricht 4 mbar), das nach $F^{19}$-Spektrum zu 40 Gew.-% aus 1-Trifluor-methylnaphthalin und zu 60 Gew.-% aus 2-Trifluor-methylnaphthalin besteht.

Beispiel 3

Analog Beispiel 1 werden 400 g 1-Naphthalincarbonsäurechlorid in 1 800 ml Tetrachlorkohlenstoff und 1 800 ml Fluorwasserstoff für 6 1/2 Stunden bei 109 °C und 17 bar umgesetzt. Nach Destillation erhält man 370 g 5-Trifluormethyl-naphthalin-1-carbonsäurefluorid ; Kp. : 132 bis 140 °C/15 mm Hg (das entspricht 20 mbar) ; Fp. : 85 bis 86 °C nach Umkristallisation aus Hexan.

Beispiel 4

Analog Beispiel 1 werden 400 g 1-Naphthalinsulfonsäurechlorid mit 1 800 ml Fluorwasserstoff und 1 600 ml Tetrachlorkohlenstoff für 8 Stunden bei 108 bis 110 °C und 17 bar umgesetzt. Durch Destillation unter vermindertem Druck erhält man 396 g eines festen Produktes, das aus 5-Trifluormethyl-1-naphthalinsulfonsäurefluorid und 8-Trifluormethyl-1-naphthalinsulfonsäurefluorid besteht. Das Verhältnis beider Verbindungen beträgt nach $F^{19}$-Spektrum 65 zu 35 Gew.-%. Reines 5-Trifluormethyl-1-naphthalinsulfonsäurefluorid hat einen Schmelzpunkt von 87 bis 88 °C und einen Siedepunkt von 140 bis 142 °C/0,2 mm Hg (das entspricht 2.66 mbar).

Beispiel 5

Analog Beispiel 1 werden 100 g 1-Nitronaphthalin in 400 ml Fluorwasserstoff und 400 ml Tetrachlorkohlenstoff für 6 Stunden bei 110 °C/18 bar zur Reaktion gebracht. Nach destillativer Aufarbeitung erhält man 90 g eines Reaktionsproduktes, das 15,7 Gew.-% 5-Trifluormethyl-1-nitronaphthalin enthält, während der Rest aus nicht-umgesetztem 1-Nitronaphthalin besteht. Das entspricht 42 % der theoretischen Ausbeute, bezogen auf umgesetztes 1-Nitronaphthalin. Das erhaltene 5-Trifluormethyl-1-nitronaphthalin hat einen Kp. : 120 °C/0,4 mm Hg (das entspricht 0.53 mbar) und einen Fp. : 107 bis 108 °C.

Beispiel 6

Analog Beispiel 1 werden 100 g 9,10-Dichloranthracen mit 400 ml Fluorwasserstoff und 500 ml Tetrachlorkohlenstoff während 6 Stunden bei 95 °C und 14,5 bar umgesetzt. Nach dem Abdestillieren des überschüssigen Fluorwasserstoffs und des überschüssigen Tetrachlorkohlenstoffs wird der Rückstand aus Ethanol umkristallisiert. Man erhält 25 g 1-Trifluormethyl-9,10-dichloranthracen vom Fp. : 142 bis 143 °C.

**Ansprüche**

1. Verfahren zur Herstellung von gegebenenfalls substituierten Trifluormethyl-naphthalinen durch Umsetzung von Naphthalinen mit Tetrachlorkohlenstoff und Fluorwasserstoff und unter Druck, dadurch gekennzeichnet, daß man eine Verbindung der Formel

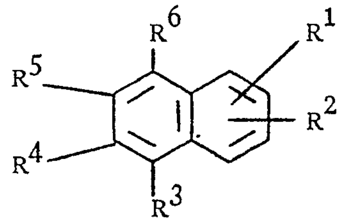

in der

$R^1$ Wasserstoff, Alkyl, Aralkyl, Aryl, Aryloxy, Arylthio, Polyhalogenalkoxy, Polyhalogenalkylthio, Halogen, Nitro, Halogencarbonyl, Halogensulfonyl, Alkylsulfonyl oder Arylsulfonyl bedeutet, wobei die in den Substituenten $R^1$ enthaltenen aromatischen Kerne ihrerseits durch Halogen, Alkyl, Polyhalogen alkoxy oder Polyhalogenalkylthio substituiert sein können,

$R^2$ für Wasserstoff, Halogen oder Alkyl steht und

$R^3$, $R^4$, $R^5$ und $R^6$ Wasserstoff bedeuten oder $R^3$ und $R^4$ oder $R^4$ und $R^5$ jeweils gemeinsam einen kondensierten aromatischen Ring bedeuten, wobei für den Fall, daß $R^3$ und $R^4$ gemeinsam einen kondensierten aromatischen Ring bilden, $R^5$ und $R^6$ unabhängig voneinander außer Wasserstoff auch noch Halogen, Nitro, Halogencarbonyl oder Halogensulfonyl bedeuten können und wobei für den Fall, daß $R^4$ und $R^5$ gemeinsam einen kondensierten aromatischen Ring bilden, $R^3$ und $R^6$ unabhängig voneinander außer Wasserstoff auch noch Halogen Nitro, Halogencarbonyl oder Halogensulfonyl bedeuten können, einsetzt, die Umsetzung unter Ausschluß von Kupfer bei einer Temperatur von 70 bis 120 °C und bei einem Druck von 5 bis 30 bar durchführt, und das Reaktionsprodukt gegebenenfalls anschließend mit katalytisch angeregtem Wasserstoff behandelt.

2. Verfahren nach Anspruch 1, dadurch gekennzeichnet, daß man ein Naphthalin der Formel

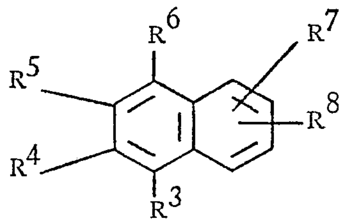

in der

$R^7$ Aryloxy, Arylthio, Polyhalogenalkoxy, Polyhalogenalkylthio, Halogen, Nitro, Halogencarbonyl, Halogensulfonyl, Alkylsulfonyl oder Arylsulfonyl bedeutet, wobei die im Substituenten $R^7$ enthaltenen aromatischen Kerne ihrerseits durch Halogen, Alkyl, Polyhalogenalkoxy oder Polyhalogenalkylthio substituiert sein können,

$R^8$ für Wasserstoff oder Halogen steht und

$R^3$ bis $R^6$ die in Anspruch 1 angegebene Bedeutung haben einsetzt.

3. Verfahren nach Anspruch 1, dadurch gekennzeichnet, daß man ein Naphthalin der Formel

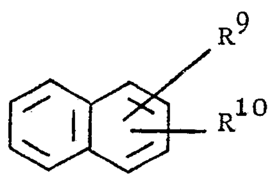

in der

$R^9$ Halogen, Nitro, Halogencarbonyl oder Halogensulfonyl bedeutet und

$R^{10}$ für Wasserstoff oder Halogen steht, einsetzt.

4. Verfahren nach Anspruch 1, dadurch gekennzeichnet, daß man ein Naphthalin der Formel

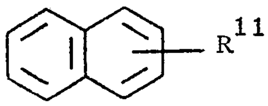

in der

$R^{11}$ Chlorcarbonyl oder Chlorsulfonyl bedeutet, einsetzt.

5. Verfahren nach Anspruch 1, dadurch gekennzeichnet, daß man eine Verbindung der Formel

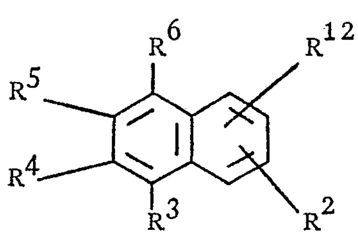

in der

$R^2$ bis $R^6$ die in Anspruch 1 genannte Bedeutung haben und

$R^{12}$ Chlor oder Brom bedeutet, einsetzt, und das Reaktionsprodukt anschließend in Gegenwart eines Raney-Katalysators und in Gegenwart einer alkalisch reagierenden Substanz mit Wasserstoff weiter umsetzt.

**Claims**

1. Process for the preparation of optionally substituted trifluoromethyl-naphtalenes by reacting naphthalenes with carbon tetrachloride and hydrogen fluoride and under pressure, characterised in that a compound of the formula

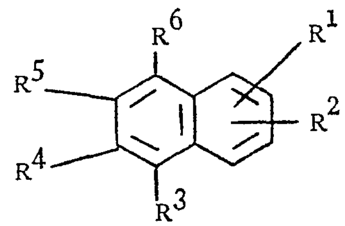

in which

$R^1$ denotes hydrogen, alkyl, aralkyl, aryl, aryloxy, arylthio, polyhalogenoalkoxy, polyhalogenoalkylthio, halogen, nitro, halogenocarbonyl, halogenosulphonyl, alkylsulphonyl or arylsulphonyl, it being possible for the aromatic nuclei contained in the substituents $R^1$ to be in turn substituted by halogen, alkyl, polyhalogenoalkoxy or polyhalogenoalkylthio,

$R^2$ represents hydrogen, halogen or alkyl and

$R^3$, $R^4$, $R^5$ and $R^6$ denote hydrogen, or $R^3$ and $R^4$ or $R^4$ and $R^5$ in each case together denote a fused-on aromatic ring, and wherein, in the case where $R^3$ and $R^4$ together form a fused-on aromatic ring, $R^5$ and $R^6$ independently of one another can also denote, in addition to hydrogen, halogen, nitro, halogenocarbonyl or halogenosulphonyl, and wherein, in the case where $R^4$ and $R^5$ together form a fused-on aromatic ring, $R^3$ and $R^6$ independently of one another can also denote, in addition to hydrogen, halogen, nitro, halogenocarbonyl of halogenosulphonyl, is employed, the reaction is carried out with the exclusion of copper at a temperature of 70 to 120 °C and under a pressure of 5 to 30 bars and the reaction product is then optionally treated with catalytically activated hydrogen.

2. Process according to Claim 1, characterised in that a naphthalene of the formula

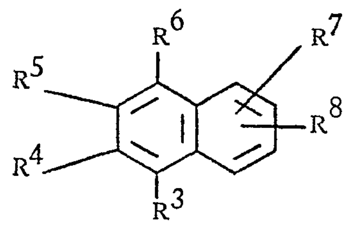

in which

$R^7$ denotes aryloxy, arylthio, polyhalogenoalkoxy, polyhalogenoalkylthio, halogen, nitro, halogenocarbonyl, halogenosulphonyl, alkylsulphonyl or arylsulphonyl, it being possible for the aromatic nuclei contained in the substituent $R^7$ to be in turn substituted by halogen, alkyl, polyhalogenoalkoxy or polyhalogenoalkylthio,

$R^8$ represents hydrogen or halogen and $R^3$ to $R^6$ have the meaning indicated in Claim 1, is employed.

3. Process according to Claim 1, characterised in that a naphthalene of the formula

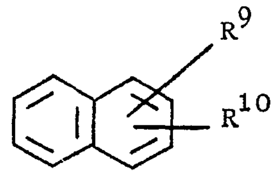

in which

$R^9$ denotes halogen, nitro, halogenocarbonyl or halogenosulphonyl and

$R^{10}$ represents hydrogen or halogen, is employed.

4. Process according to Claim 1, characterised in that a naphthalene of the formula

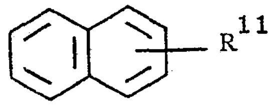

in which

$R^{11}$ denotes chlorocarbonyl or chlorosulphonyl, is employed.

5. Process according to Claim 1, characterised in that a compound of the formula

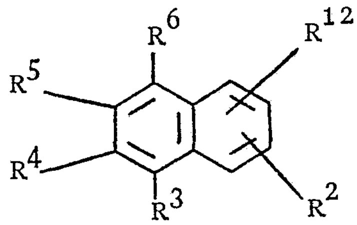

in which

$R^2$ to $R^6$ have the meaning given in Claim 1 and

$R^{12}$ denotes chlorine or bromine, is employed, and the reaction product is then reacted further with hydrogen in the presence of a Raney catalyst and in the presence of an alkaline substance.

**Revendications**

1. Procédé de préparation de trifluorométhyl-naphtalènes éventuellement substitués par réaction de naphtalènes avec du tétrachlorure de carbone et de l'acide fluorhydrique en travaillant sous pression, caractérisé en ce qu'on utilise un composé de formule

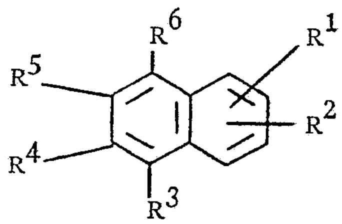

dans laquelle

$R^1$ représente un atome d'hydrogène, un groupe alkyle, un groupe aralkyle, un groupe aryle, un groupe aryloxy, un groupe arylthio, un groupe polyhalogénalcoxy, un groupe polyhalogénalkylthio, un atome d'halogène, un groupe nitro, un groupe halogénocarbonyle, un groupe halogénosulfonyle, un groupe alkylsulfonyle ou un groupe arylsulfonyle, les noyaux aromatiques contenus dans les substituants $R^1$ pouvant, à leur tour, être substitués par un atome d'halogène, un groupe alkyle, un groupe polyhalogénalcoxy ou par un groupe polyhalogénalkylthio,

$R^2$ représente un atome d'hydrogène, un atome d'halogène ou un groupe alkyle, et

$R^3$, $R^4$, $R^5$ et $R^6$ représentent chacun un atome d'hydrogène ou $R^3$ et $R^4$ ou $R^4$ et $R^5$ ensemble représentent chaque fois un noyau aromatique condensé et, si $R^3$ et $R^4$ ensemble forment un noyau aromatique condensé, $R^5$ et $R^6$ peuvent représenter, indépendamment l'un de l'autre et outre un atome d'hydrogène, également un atome d'halogène, un groupe nitro, un groupe halogénocarbonyle ou un groupe halogénosulfonyle et, si $R^4$ et $R^5$ ensemble forment un noyau aromatique condensé, $R^3$ et $R^6$ peuvent représenter indépendamment l'un de l'autre, outre un atome d'hydrogène, également un atome d'halogène, un groupe nitro, un groupe halogénocarbonyle ou un groupe halogénosulfonyle, on effectue la réaction en absence de cuivre à une température de 70 à 120 °C et sous une pression de 5 à 30 bars et ensuite, on traite éventuellement le produit réactionnel avec de l'hydrogène activé catalytiquement.

2. Procédé suivant la revendication 1, caractérisé en ce qu'on utilise un naphtalène de formule

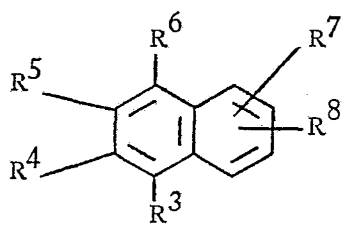

dans laquelle

$R^7$ représente un groupe aryloxy, un groupe arylthio, un groupe polyhalogénalcoxy, un groupe polyhalogénalkylthio, un atome d'halogène, un groupe nitro, un groupe halogénocarbonyle, un groupe halogénosulfonyle, un groupe alkylsulfonyle ou un groupe arylsulfonyle, les noyaux aromatiques contenus dans les substituants $R^7$ pouvant, à leur tour, être substitués par un atome d'halogène, un groupe alkyle, un groupe polyhalogénalcoxy ou par un groupe polyhalogénalkylthio,

$R^8$ représente un atome d'hydrogène ou un atome d'halogène, et

$R^3$ à $R^6$ ont les significations indiquées dans la revendication 1.

3. Procédé suivant la revendication 1, caractérisé en ce qu'on utilise un naphtalène de formule

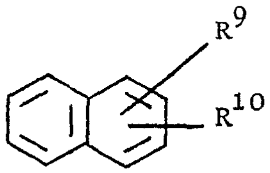

dans laquelle

$R^9$ représente un atome d'halogène, un groupe nitro, un groupe halogénocarbonyle ou un groupe halogénosulfonyle, et

$R^{10}$ représente un atome d'hydrogène ou un atome d'halogène.

4. Procédé suivant la revendication 1, caractérisé en ce qu'on utilise un naphtalène de formule dans laquelle

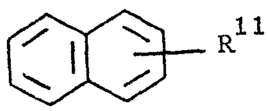

$R^{11}$ représente un groupe chlorocarbonyle ou un groupe chlorosulfonyle.

5. Procédé suivant la revendication 1, caractérisé en ce qu'on utilise un composé de formule

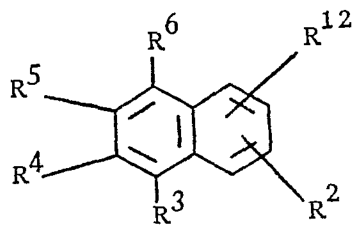

dans laquelle

$R^2$ à $R^6$ ont les significations indiquées dans la revendication 1, et

$R^{12}$ représente un atome de chlore ou un atome de brome, puis on soumet le produit réactionnel à une réaction complémentaire avec de l'hydrogène en présence d'un catalyseur de Raney et en présence d'une substance à réaction alcaline.